# EUROPEAN PATENT APPLICATION

(11) **EP 4 331 599 A1**
(43) Date of publication of application: **06.03.2024**
(21) Application number: 21938973.1
(22) Date of filing: 19.11.2021
(51) Int. Cl.: A61K 38/17, A61P 11/00, A61P 29/00

(54) **MEDICATION FOR TREATING LUNG INJURY**

(30) Priority: 25.04.2021 CN 202110448591
(71) Applicant: Shanghai Seme Cell Technology Co., Ltd, Shanghai 200241 (CN)
(72) Inventor: HOU, Xifan, Shanghai 200241 (CN); ZHANG, Wenjie, Shanghai 200241 (CN); WANG, Bin, Shanghai 200241 (CN)
(74) Representative: Elzaburu S.L.P.
(86) International application number: PCT/CN2021/131749
(87) International publication number: WO 2022/227513

(57) **Abstract**

The present invention relates to a medication for treating a lung injury. Specifically, the present invention provides uses of an annexin in preparation of a composition or a formulation. The composition or the formulation has one or more uses selected from the following group consisting of: (i) preventing and/or treating a lung injury; and (ii) reducing neutrophil aggregation.

## Description

### TECHNIAL FIELD

The present invention relates to the field of medicine, in particular, to a medication for treating lung injury.

### BACKGROUND

Acute lung injury (ALI) and acute respiratory distress syndrome (ARDS) seriously affect human health. ARDS refers to a clinical syndrome mainly manifested as an alveolar capillary injury that occurs after an intrapulmonary or extrapulmonary attack such as severe infection, trauma, or shock. ARDS belongs to a severe stage or type of ALI. Clinical features of ARDS include respiratory rate and distress, progressive hypoxemia, and X-ray diffuse alveolar infiltration. However, it lacks medications for effective treatment of ALI and ARDS in clinical at present.

Therefore, there is still a need for developing a medication for effectively treating lung injury such as ALI and ARDS in the field.

### SUMMARY OF THE INVENTION

The purpose of the present invention is to provide uses of an annexin in effective treatment of lung injuries such as ALI and ARDS.

A first aspect of the present invention provides uses of an annexin in preparation of a composition or a formulation, the composition or formulation has one or more uses selected from the following group consisting of: (i) preventing and/or treating lung injury; and (ii) reducing a quantity of neutrophils.

In another preferred example, the composition or formulation further has one or more uses selected from the following group consisting of: increasing blood gas PO₂ and/or sO₂% indicators; reducing pulmonary inflammation; reducing inflammatory factors in serum; improving the degree of lung lesions; and/or decreasing a wet/dry ratio of lung.

In another preferred example, the annexin includes an annexin A5.

In another preferred example, the lung injury is selected from the following group consisting of: an acute lung injury, a respiratory distress syndrome, or a combination thereof.

In another preferred example, the respiratory distress syndrome includes an acute respiratory distress syndrome.

In another preferred example, the respiratory distress syndrome includes a neonatal respiratory distress syndrome.

In another preferred example, the lung injury includes a lung injury caused by an increase in neutrophils.

In another preferred example, the lung injury includes a lung injury caused by a cytokine storm.

In another preferred example, the preventing and/or treating lung injury includes preventing and/or treating lung injury by reducing quantity of neutrophils.

In another preferred example, the preventing and/or treating lung injury includes preventing and/or treating lung injury by improving in the cytokine storm.

In another preferred example, the improving in the cytokine storm includes reducing, inhibiting, or eliminating.

In another preferred example, the preventing and/or treating lung injury includes preventing and/or treating lung injury by reducing quantity of neutrophils and/or inhibiting the cytokine storm caused by the increase in quantity of neutrophils.

In another preferred example, the neutrophils include neutrophils in lung.

In another preferred example, the cytokine storm includes a cytokine storm in lung.

In another preferred example, the neutrophils include neutrophils in alveoli.

In another preferred example, the cytokine storm includes a cytokine storm in the alveoli.

In another preferred example, the "reducing quantity of neutrophils" refers to reducing quantity of neutrophils in a subject or reducing neutrophil aggregation in lung of a subject.

In another preferred example, the subject is a human or a non-human mammal (such as a rodent).

In another preferred example, the subject is a human with an increase in quantity of neutrophils.

In another preferred example, the "increase in the quantity of neutrophils" refers to a higher quantity of neutrophils A1 in a lung injury subject, compared with a quantity of neutrophils A0 in a normal subject; and preferably, A1/A0 ≥ 1.1, preferably ≥ 1.2, more preferably ≥ 1.3, more preferably ≥ 1.4, more preferably ≥ 1.5, more preferably ≥ 1.6, more preferably ≥ 1.8, more preferably ≥ 2, more preferably ≥ 3, more preferably ≥ 5, more preferably ≥ 8.

In another preferred example, the subject is a lung injury subject.

In another preferred example, the subject is a subject who received, is receiving, or will receive treatment of lung injury.

In another preferred example, the "reducing quantity of neutrophils" refers to reducing quantity of neutrophils C1 in lung (such as alveoli) compared with a quantity of neutrophils C0 in a control.

In another preferred example, the quantity of neutrophils C0 in control is a quantity of neutrophils Czs in the subject before therapy or treatment using the annexin; or a quantity of neutrophils Cdz in a control group.

In another preferred example, the control group includes quantity of neutrophils in normal subject.

In another preferred example, the "reducing quantity of neutrophils" refers to C1/Czs ≤ 0.9, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1.

In another preferred example, the "reducing quantity of neutrophils" refers to C1/Cdz ≤ 0.9, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1.

In another preferred example, the lung injury have one or more of the following features:
(a) a low survival rate;
(b) a decrease in blood gas PO₂ and/or sO₂% indicators;
(c) presence of inflammation in lung;
(d) presence of inflammatory factors in serum;
(e) lung lesions;
(f) a high wet/dry ratio of lung.

In another preferred example, the "low survival rate" refers to a survival rate of lung injury subject as low as 70%, and preferably 60%, 50%, 40%, 30%, 20%, 10%, or 5%.

In another preferred example, the "decrease in PO₂" refers to a lower PO₂ (LB1) of the lung injury subject, compared with a PO₂ (LB0) of the normal subject; and preferably LB0/LB1 ≥ 1.1, preferably ≥ 1.2, more preferably ≥ 1.3, more preferably ≥ 1.4, more preferably ≥ 1.5, more preferably ≥ 1.6, more preferably ≥ 1.8, more preferably ≥ 2, more preferably ≥ 3, more preferably ≥ 5, more preferably ≥ 10.

In another preferred example, the "decrease in sO₂%" refers to a lower sO₂% (LD1) of the lung injury subject, compared with a sO₂% (LD0) of the normal subject; and preferably LD0/LD1 & 1.1, preferably & 1.15, more preferably & 1.2, more preferably & 1.25, more preferably & 1.3, more preferably & 1.4, more preferably & 1.5, more preferably & 1.8, more preferably & 2, more preferably & 3, more preferably & 5.

In another preferred example, the presence of inflammation in the lung includes one or more features selected from the following group consisting of:
(c-1) an increase in pulmonary inflammatory factors;
(c-2) an increase in pulmonary white blood cells;
(c-3) an increase in pulmonary total proteins.

In another preferred example, the pulmonary inflammatory factors include TNF-α, IL-10, IL-6, and/or IL-1β.

In another preferred example, the pulmonary white blood cells include WBC (white blood cells), Neut (neutrophils), Lymph (lymphocytes), and Mono (monocytes).

In another preferred example, the "increase in pulmonary inflammatory factors" refers to a higher quantity of pulmonary inflammatory factors (LE1) of the lung injury subject, compared with a quantity of inflammatory factors (LE0) of the normal subject; and preferably, LE1/LE0 & 5, more preferably & 10, more preferably & 20, more preferably & 30, more preferably & 50, more preferably & 100, more preferably & 200, more preferably & 300, more preferably & 500, more preferably & 1000.

In another preferred example, the "increase in white blood cells" refers to a higher quantity of white blood cells (LF1) of the lung injury subject, compared with a quantity of white blood cells (LF0) of the normal subject; and preferably, LF1/LF0 ≥ 1.1, preferably ≥ 1.2, more preferably ≥ 1.5, more preferably ≥ 2, more preferably ≥ 3, more preferably ≥ 5, more preferably ≥ 10, more preferably ≥ 20, more preferably ≥ 30, more preferably ≥ 50, more preferably ≥ 100.

In another preferred example, the "increase in pulmonary total proteins" refers to a higher quantity of total proteins (LH1) of the lung injury subject, compared with a quantity of total proteins (LH0) of the normal subject; and preferably LH1/LH0 ≥ 1.1, preferably ≥ 1.2, more preferably ≥ 1.5, more preferably ≥ 2, more preferably ≥ 3, more preferably ≥ 5, more preferably ≥ 10, more preferably ≥ 20, more preferably ≥ 30, more preferably ≥ 50, more preferably ≥ 100.

In another preferred example, the inflammatory factors in the serum include TNF-α, IL-10, IL-6, and/or IL-1β.

In another preferred example, the "presence of inflammatory factors in serum" refers to a higher quantity of inflammatory factors (LJ1) in serum of the lung injury subject, compared with a quantity of inflammatory factors (LJ0) of normal subject; and preferably, LJ1/LJ0 & 2, preferably & 5, more preferably & 10, more preferably & 20, more preferably & 30, more preferably & 50, more preferably & 100, more preferably & 200, more preferably & 300, more preferably & 500, more preferably & 1000.

In another preferred example, the "high wet/dry ratio of the lung" refers to a higher wet/dry ratio (LK1) of the lung injury subject, compared with a wet/dry ratio (LK0) of normal subject; and preferably, LK1/LK0 ≥ 1.1, preferably ≥ 1.2, more preferably ≥ 1.3, more preferably ≥ 1.4, more preferably ≥ 1.5, more preferably ≥ 1.6, more preferably ≥ 1.8, more preferably ≥ 2, more preferably ≥ 3, more preferably ≥ 5, more preferably ≥ 10.

In another preferred example, the preventing and/or treating lung injuries include one or more features selected from the following group consisting of:
(i) increasing survival rate;
(ii) increasing blood gas PO₂ and/or sO₂% indicators;
(iii) reducing pulmonary inflammation;
(iv) reducing inflammatory factors in serum;
(v) improving degree of lung lesions;
(vi) decreasing wet/dry ratio of lung.

In another preferred example, the "increasing survival rate" refers to increasing a survival rate of an administered subject to 70%, and preferably 80%, 85%, 90%, 92%, 95%, 99%, or 99.5%.

In another preferred example, the "increasing PO₂" refers to a higher PO₂ (B1) of the administered subject, compared with the PO₂ (B0) of the lung injury subject; and preferably, B1/B0 ≥ 1.1, preferably ≥ 1.2, more preferably ≥ 1.3, more preferably ≥ 1.4, more preferably ≥ 1.5, more preferably ≥ 1.6, more preferably ≥ 1.8, more preferably ≥ 2, more preferably ≥ 3, more preferably ≥ 5, more preferably ≥ 10.

In another preferred example, the "increasing sO₂%" refers to a higher sO₂% (D1) of the administered subject, compared with the sO₂% (D0) of the lung injury subject; and preferably D1/D0 ≥ 1.1, preferably ≥ 1.2, more preferably ≥ 1.3, more preferably ≥ 1.4, more preferably ≥ 1.5, more preferably ≥ 1.6, more preferably ≥ 1.8, more preferably ≥ 2, more preferably ≥ 3, more preferably ≥ 5, more preferably ≥ 10.

In another preferred example, the reducing the pulmonary inflammation is performed by one or more ways selected from the following group consisting of:
(iii-1) reducing pulmonary inflammatory factors;
(iii-2) reducing pulmonary white blood cells;
(iii-3) reducing pulmonary total proteins.

In another preferred example, the pulmonary inflammatory factors include TNF-α, IL-10, IL-6, and/or IL-1β.

In another preferred example, the pulmonary white blood cells include WBC (white blood cells), Neut (neutrophils), Lymph (lymphocytes), and Mono (monocytes).

In another preferred example, the "reducing pulmonary inflammatory factors" refers to a lower quantity of pulmonary inflammatory factors (E1) of the administrated subject than quantity of inflammatory factors (E0) of the lung injury subject; and preferably E0/E1 & 1.1, preferably & 1.2, more preferably & 1.5, more preferably & 2, more preferably & 3, more preferably & 5, more preferably & 10, more preferably & 20, more preferably & 30, more preferably & 50, more preferably & 100.

In another preferred example, the "reducing white blood cells" refers to a lower quantity of white blood cells (F1) of the administrated subject than quantity of white blood cells (F0) of the lung injury subject; and preferably, F0/F1 & 1.1, preferably & 1.2, more preferably & 1.5, more preferably & 2, more preferably & 3, more preferably & 5, more preferably & 10, more preferably & 20, more preferably & 30, more preferably & 50, more preferably & 100.

In another preferred example, the "reducing pulmonary total proteins" refers to a lower quantity of total proteins (H1) of the administered subject than quantity of total proteins (H0) of the lung injury subject; and preferably, H0/H1 ≥ 1.1, preferably ≥ 1.2, more preferably ≥ 1.3, more preferably ≥ 1.4, more preferably ≥ 1.5, more preferably ≥ 1.6, more preferably ≥ 1.8, more preferably ≥ 2, more preferably ≥ 3, more preferably ≥ 5, more preferably ≥ 10.

In another preferred example, the inflammatory factors in the serum include TNF-α, IL-10, IL-6, and/or IL-1β.

In another preferred example, the "reducing inflammatory factors in the serum" refers to a lower quantity of inflammatory factors (J1) in serum of the administrated subject than quantity of inflammatory factors (10) in the lung injury subject; and preferably J0/J1 ≥ 2, preferably ≥ 5, more preferably ≥ 10, more preferably ≥ 20, more preferably ≥ 30, more preferably ≥ 50, more preferably ≥ 100, more preferably ≥ 200, more preferably ≥ 300, more preferably ≥ 500, more preferably ≥ 1000.

In another preferred example, the "reducing wet/dry ratio of the lung" refers to a lower wet/dry ratio (K1) of the administered subject than the wet/dry ratio (K0) of the lung injury subject; and preferably, K0/K1 ≥ 1.1, preferably ≥ 1.2, more preferably ≥ 1.3, more preferably ≥ 1.4, more preferably ≥ 1.5, more preferably ≥ 1.6, more preferably ≥ 1.8, more preferably ≥ 2, more preferably ≥ 3, more preferably ≥ 5, more preferably ≥ 10.

In another preferred example, the administrated subject refers to a subject who has been treated using the annexin.

In another preferred example, the composition or formulation includes a pharmaceutical composition or formulation, a food composition or formulation, a health product composition or formulation, or a dietary supplement.

In another preferred example, the composition or formulation further includes a pharmaceutically acceptable carrier for food, a health product, or a diet.

In another preferred example, the composition or formulation further includes other medications for preventing and/or treating lung injury;
Preferably, the other medications for preventing and/or treating lung injuries are selected from the following group consisting of: a glucocorticoid, a vitamin, or a combination thereof.

In another preferred example, the vitamin is selected from the following group consisting of: vitamin C, vitamin E, or a combination thereof.

In another preferred example, the dosage form of the composition or formulation is an oral formulation, an external formulation, or an injection formulation.

In another preferred example, the dosage form of the composition or formulation is a solid formulation, a liquid formulation, or a semi-solid formulation.

In another preferred example, the dosage form of the composition or formulation is powder, granules, capsules, injection, tincture, oral liquid, tablets, buccal tablet, dry powder inhalation, aerosol, or spray.

In another preferred example, the dosage form of the composition or formulation is a respiratory administration formulation.

In another preferred example, the dosage form of the composition or formulation is an injection.

In another preferred example, the injection is an intravenous injection, an intramuscular injection, a subcutaneous formulation, or a respiratory administration formulation.

In another preferred example, the respiratory administration formulation is an airway nebulizer formulation.

In another preferred example, in the composition or formulation, the annexin has a mass percentage of 5 wt%, preferably 1-20 wt%, based on a total weight of the composition or formulation.

A second aspect of the present invention provides a method for (i) preventing and/or treating lung injury and/or (ii) reducing a quantity of neutrophils, where the method includes administering an annexin to a subject.

In another preferred example, the non-human mammal includes pigs, cows, sheep, rats, mice, or rabbits.

In another preferred example, an administered dose is 0.2-5 mg/kg, such as 0.2 mg/kg, 0.5 mg/kg, 0.75 mg/kg, or 1 mg/kg.

It should be understood that the foregoing technical features of the present invention and specific technical features described below (such as in examples) can be combined with each other to form new or preferred technical solutions within the scope of the present invention, which will not be detailed here due to space limitations.

### BRIEF DESCRIPTION OF DRAWINGS

FIG. 1 shows a count of neutrophils in an alveolar lavage fluid in Example 1, *p < 0.05, G2-G12 VS G1, using One-way ANOVA/Dunnett's.
FIG. 2 shows a percentage of neutrophils in the alveolar lavage fluid in Example 1, *p < 0.05, **p < 0.01, ***p < 0.001, G2-G12 VS G1, using One-way ANOVA/Dunnett's.
FIG. 3 shows pulmonary pathology scores in Example 1.
FIG. 4 shows staining of pathological sections of lung tissues in Example 1.
FIG. 5 shows effects of the annexin on blood gas analysis of rats with lung injuries in Example 2 (*x̅*±*SD*).
FIG. 6 shows effects of the annexin on a total number and classification of white blood cells in a bronchoalveolar lavage fluid (BALF) of rats with lung injuries in Example 2 (*x̅*±*SD).*
FIG. 7 shows effects of the annexin on total proteins in BALF of rats with lung injuries in Example 2 (*x̅*±*SD*).
FIG. 8 shows effects of the annexin on inflammatory factors in BALF of rats with lung injuries in Example 2 (*x̅*±*SD*).
FIG. 9 shows effects of the annexin on inflammatory factors in serum of rats with lung injuries in Example 2 (*x̅*±*SD*).
FIG. 10 shows effects of the annexin on the degree of tissue lesions of rats with lung injuries in Example 2.
FIG. 11 shows effects of the annexin on a wet/dry ratio of rats with lung injuries in Example 2 (*x̅*±*SD*).

### DETAILED DESCRIPTION OF EMBODIMENTS

After extensive and in-depth researches, the inventor found for the first time that an annexin can be used for preventing and/or treating lung injuries and reducing a quantity of neutrophils, and can also significantly reduce deaths caused by lung injuries, increase blood gas PO₂ and/or sO₂% indicators, reduce pulmonary inflammation, reduce an increase in inflammatory factors in serum caused by lung injuries, improve the degree of lung lesions, and reduce a wet/dry ratio of the lung. On this basis, the present invention is completed.

### Term

Unless otherwise defined, all technical and scientific terms herein have the same meanings as those commonly understood by ordinary technical personnel in the art to which the present invention belongs.

As used herein, the terms "include", "comprise", and "contain" are interchangeable and include not only open definitions, but also-semi closed and closed definitions. That is, the described terms include "consist of" and "substantially consist of".

### Annexin

Annexin is a type of widely distributed calcium-dependent phospholipid binding proteins. Based on molecular phylogenetics and comparative genomics analysis, the protein family is uniformly named annexin (abbreviated as Anx), followed by capital letters to represent different species and genera: A, B, C, D, and E represent vertebrates, invertebrates, fungi and slime worms, plants, and protozoa, respectively. As of now, the Anx A family has a total of 12 members: A1-A11 and A13 (A12 not specified).

Representatively, the annexin includes an annexin A5. The annexin A5 (AnxA5) is widely distributed in various tissues and cells of bodies. In vitro studies found that, the AnxA5 has various functions such as anti-phospholipase, anticoagulation, and anti-kinase. However, in vivo functions thereof are not very clear, and now it is mainly used as a reagent for detecting cell apoptosis.

The annexin that can be used for the present invention is not particularly limited, and may be derived from any organism, preferably from mammals (such as primates), more preferably from a human. Moreover, it should be understood that, the "annexin" includes a wild-type or mutant-type (including truncated) annexin, as long as the mutant-type annexin retains or maintains detoxification activity of the wild-type annexin.

### Use

The present invention provides uses of an annexin in preparation of a composition or formulation. The composition or formulation has one or more uses selected from the following group consisting of: (i) preventing and/or treating lung injury; (ii) reducing a quantity of neutrophils.

The lung injury described in the present invention are not particularly limited. Preferably, the lung injuries include, butare not limited to, an acute lung injury, a respiratory distress syndrome, or a combination thereof.

Representatively, the lung injury include the acute lung injury.

Representatively, the lung injury include the respiratory distress syndrome.

The respiratory distress syndrome described in the present invention is preferably an acute respiratory distress syndrome.

Multiple reasons may cause lung injury, such as direct injury, shock, pancreatitis, inhalation of smoke and toxic gas, pneumonia, or reperfusion injury. Researches of the present invention show that a "cytokine storm" is an important cause of lung injury. Neutrophils produce a lot of cytokines such as TNF-α, IL-1, and IL-6 (namely, cytokine storms) when the quantity of neutrophils in the lung increases. These cytokines attack tissues and cause the acute lung injury and an organ failure. Therefore, the cytokine storms can be improved by reducing the quantity of neutrophils in the lung to prevent and/or treat the lung injuries.

In a preferred example of the present invention, the lung injury includes a lung injury caused by an increase in neutrophils.

In a preferred example of the present invention, the lung injury includes a lung injury caused by a cytokine storm.

In a preferred example of the present invention, the preventing and/or treating lung injury includes preventing and/or treating the lung injuries by reducing the quantity of neutrophils.

In a preferred example of the present invention, the preventing and/or treating lung injury includes preventing and/or treating the lung injury by improving in the cytokine storm.

In another preferred example, the improving in the cytokine storm includes reducing, inhibiting, or eliminating.

In a preferred example of the present invention, the preventing and/or treating lung injury includes preventing and/or treating the lung injury by reducing the quantity of neutrophils and/or inhibiting the cytokine storm caused by the increase in the quantity of neutrophils.

In a preferred example of the present invention, the neutrophils include neutrophils in the lung.

In another preferred example, the neutrophils include neutrophils in alveoli.

In a preferred example of the present invention, the cytokine storm includes a cytokine storm in the lung.

In another preferred example, the cytokine storm includes a cytokine storm in the alveoli.

In a preferred example of the present invention, the "reducing quantity of neutrophils" refers to reducing the quantity of neutrophils in a subject.

In another preferred example, the subject is a human or a non-human mammal (such as a rodent).

In a preferred example of the present invention, the subject is a human with an increase in quantity of neutrophils.

In another preferred example, the "increase in quantity of neutrophils" refers to a higher quantity of neutrophils A1 of a lung injury subject, compared with a quantity of neutrophils A0 of a normal subject; and preferably, A1/A0 ≥ 1.1, preferably ≥ 1.2, more preferably ≥ 1.3, more preferably ≥ 1.4, more preferably ≥ 1.5, more preferably ≥ 1.6, more preferably≥ 1.8, more preferably ≥ 2, more preferably≥ 3, more preferably ≥ 5, more preferably ≥ 8.

In another preferred example, the subject is a lung injury subject.

In another preferred example, the lung injury subject is a subject who received, is receiving, or will receive treatment of lung injury.

In a preferred example of the present invention, the "reducing quantity of neutrophils" refers to reducing quantity of neutrophils C1 in the lung (such as alveoli) compared to a quantity of neutrophils C0 in a control.

In another preferred example, the quantity of neutrophils C0 in the control is a quantity of neutrophils Czs in the subject before therapy or treatment using the annexin; or a quantity of neutrophils Cdz in a control group.

In another preferred example, the control group includes the quantity of neutrophils in the normal subject.

In another preferred example, the "reducing quantity of neutrophils" refers to C1/Czs ≤ 0.9, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1.

In another preferred example, the "reducing quantity of neutrophils" refers to C1/Cdz ≤ 0.9, preferably ≤ 0.8, more preferably ≤ 0.7, more preferably ≤ 0.6, more preferably ≤ 0.5, more preferably ≤ 0.4, more preferably ≤ 0.3, more preferably ≤ 0.2, more preferably ≤ 0.1.

In another preferred example, the composition or the formulation includes a pharmaceutical composition or formulation, a food composition or formulation, a health product composition or formulation, or a dietary supplement.

In a preferred example of the present invention, the composition or formulation further includes other medications for preventing and/or treating lung injuries.

In another preferred example, the other medications for preventing and/or treating lung injuries are selected from the following group consisting of: a glucocorticoid, a vitamin, or a combination thereof.

In another preferred example, the vitamin is selected from the following group consisting of: vitamin C, vitamin E, or a combination thereof.

### Composition and administration

The composition described in the present invention includes (but is not limited to): a pharmaceutical composition, a food composition, a health-care composition, a dietary supplement, and the like.

Representatively, a cell-free fat extract of the present invention may be prepared into a pharmaceutical composition, such as tablets, capsules, powders, microparticles, solution, pastille, gel, cream, spirits, suspension, tincture, cataplasm, liniment, lotion, and aerosol. The pharmaceutical composition can be prepared by a commonly known preparation technology, and suitable medicated additives can be added to the medication.

The composition of the present invention may further include a pharmaceutically acceptable carrier for a medicine, food, a health-care product, or a diet. The "pharmaceutically acceptable carrier for a medicine, food, a health-care product, or a diet" refers to one or more compatible solid or liquid fillers or gel substances, which are suitable for human use and must have enough purity and enough low toxicity. The "compatible" indicates that each ingredient in the composition can be blended with the compound of the present invention, and the ingredients can be blended with each other without significantly reducing the efficacy of the compound. Examples of the pharmaceutically acceptable carrier for a medicine, food, a health-care product include cellulose and derivatives thereof (such as carboxymethylcellulose sodium, ethyl cellulose sodium, and cellulose acetate), gelatin, talc, solid lubricants (such as stearic acid and magnesium stearate), calcium sulfate, vegetable oils (such as soybean oil, sesame oil, peanut oil, and olive oil), polyols (such as propylene glycol, glycerol, mannitol, and sorbitol), emulsifiers (such as Tween^{®}), wetting agents (such as sodium dodecyl sulfate), colorants, flavorings, stabilizers, antioxidants, preservatives, athermal raw water, and the like.

Routes of administration of the composition of the present invention are not particularly limited. Representative administration routes include (but are not limited to) oral administration, parenteral (intravenous and intramuscular) administration, and local administration. The preferred administration routes include oral administration and injection.

The composition or formulation described in the present invention is indosage forms of an oral formulation, an external formulation, or an injection formulation. Representatively, solid dosage forms used for oral administration or administration include capsules, tablets, pills, powders, and granules. In these solid forms, the active compound is blended with at least one conventionally inert excipient (or carrier), such as sodium citrate or dicalcium phosphate, or with the following ingredients: (a) fillers or compatibilizers, such as starch, lactose, sucrose, glucose, mannitol, and silicic acid; (b) adhesives, such as hydroxymethyl cellulose, alginate, gelatin, polyvinyl pyrrolidone, sucrose, and arabic gum; (c) moisturizing agents, such as glycerol; (d) disintegrating agents, such as agar, calcium carbonate, potato starch or cassava starch, alginic acid, some composite silicates, and sodium carbonate; (e) slow solvents, such as paraffin; (f) absorption accelerators, such as quaternary amine compounds; (g) wetting agents, such as cetanol and monostearate glycerides; (b) adsorbents, such as kaolin; and (i) lubricants, such as talc, calcium stearate, magnesium stearate, solid polyethylene glycol, sodium dodecyl sulfate, or mixtures thereof. The dosage form may further include a buffer among the capsules, tablets, and pills.

The solid dosage forms such as tablets, sugar pills, capsules, pills, and granules can be prepared by coating and shell materials, such as casings and other well-known materials in the art. The solid dosage forms may include opaque agents.

Liquid dosage forms for oral administration or administration include pharmaceutically acceptable lotions, solutions, suspensions, syrups, or tinctures. In addition to active compounds, the liquid dosage forms may include inert diluents commonly used in the art, such as water or other solvents, solubilizers, and emulsifiers, such as ethanol, isopropanol, ethyl carbonate, ethyl acetate, propylene glycol, 1,3-butanediol, dimethylformamide, and oil, especially cottonseed oil, peanut oil, corn germ oil, olive oil, castor oil, and sesame oil or mixtures of these substances.

In addition to these inert diluents, the composition may further include additives such as wetting agents, emulsifiers, suspensions, sweeteners, flavoring agents, and fragrances.

In addition to the active ingredients, the suspension may include suspension agents, such as ethoxylated isooctadecanol, polyoxyethylene sorbitol, dehydrated sorbitol esters, microcrystalline cellulose, aluminum methoxide and agar, or mixtures of these substances.

The composition for parenteral injection may include physiologically acceptable sterile aqueous or anhydrous solutions, dispersions, suspensions or lotions, and sterile powders for re-dissolution into sterile injectable solutions or dispersions. Suitable aqueous and nonaqueous carriers, diluents, solvents, or excipients include water, ethanol, polyols, and suitable mixtures thereof.

Dosage forms of the compounds of the present invention for local administration or administration include ointments, powders, patches, sprays, and inhalants. The active ingredients are mixed under sterile conditions with a physiologically acceptable carrier and any preservative, buffer, or propellant if necessary.

The cell-free fat extract of the present invention can be applied or administered alone or in combination with other medications for preventing and/or treating non-proliferative scars.

When the composition is administered, a safe and effective amount of the cell-free fat extract in the present invention is applied to humans or non-human animals (such as rats, mice, dogs, cats, cows, sheep, chickens, and ducks) in need of treatment, where the applied dosage is an acceptable and effective amount in terms of pharmacy, food, or health products. As used herein, the term "safe and effective amount" refers to a dosage that produces a function or activity in a human and/or animal, and is acceptable to the human and/or animal. Those skilled in the art should understand that the "safe and effective amount" may vary with a form of a medication composition, an administration route, excipients of a medication used, severity of a disease, and combined use with other medications. For example, for a human weighing 60 kg, a daily dosage is usually 0.1-1000 mg, preferably 1-600 mg, and more preferably 2-300 mg. Of course, a specific dosage should also consider factors such as the administration route and patient's health status, which are within a skill scope of a skilled physician.

### Main advantages of the present invention include

1) The inventor found for the first time that an annexin can be used for preventing and/or treating lung injuries and reducing a quantity of neutrophils.
2) The inventor found for the first time that the annexin can comprehensively prevent and/or treat lung injuries, including increasing a survival rate; increasing blood gas PO₂ and/or sO₂% indicators; reducing pulmonary inflammation; reducing inflammatory factors in serum; improving the degree of lung lesions; and/or decreasing a wet/dry ratio of the lung.
3) The annexin can significantly reduce quantities of white blood cells and inflammatory factors in the lung and inflammatory factors in the serum, and can reduce the above indicators of diseased subjects to the levels of normal subjects.

The present invention will be further explained below in conjunction with specific examples. It should be understood that these examples are merely used for illustrating the present invention, rather than limiting the scope of the present invention. Experimental methods which are not marked with specific conditions in the following examples are usually performed under conventional conditions or conditions recommended by manufacturers. Unless otherwise specified, percentages and portions are calculated by weight.

### Examples

Annexin A5: extracted and purified from human fat tissues, Accession number: P08758.

### Example 1

### 1. Test content

### 1.1 Preparation of model

Normal adult male SD rats were randomly grouped, with 5 rats in each group. The animals were intraperitoneally injected and nebulized in the trachea with lipopolysaccharides (LPS) as a modeling reagent, as shown in Table 1:

**Table 1 Conditions for building model**

| Group Number | Group | Modeling dose ( mg/kg. body weight) |
|---|---|---|
| G0 | Normal control group | - |
| G1 | Model control group | 0.8+5 |
| G2 | Administration group | 0.8+5 |

### Modeling method:

The animals in groups G1 and G2 were intraperitoneally injected with lipopolysaccharides (0.8 mg/kg. body weight) based on their body weight, anesthetized by inhaling isoflurane after 16 h of the intraperitoneal injection, and nebulized with lipopolysaccharides (5 mg/kg. body weight) in the trachea. Group G0 was a normal control group, not administered with lipopolysaccharides, and had a normal diet. The rats in G1 and G2 showed clinical symptoms of an acute respiratory distress syndrome after treatments using lipopolysaccharides:
Intraperitoneal injection with lipopolysaccharides (LPS): Based on the body weights of the animals, each animal was administered with lipopolysaccharides by intraperitoneal injection with a disposable micro syringe.

Intratracheal nebulization with lipopolysaccharides (LPS): Each animal was anesthetized by inhaling isoflurane and then fixed on a rat fixator placed at a 45° angle. The root of the animal's tongue was pressed by a small animal anesthesia laryngoscope to expose the glottis, a pulmonary micro liquid nebulizer needle (blunt) that extracted a quantitative solution of LPS was gently inserted into the trachea, then the piston was quickly pushed to atomize the LPS solution into the lung, the needle was quickly pulled out. And each animal was taken down from the fixator, and rotated left and right with its head facing upwards to distribute LPS as evenly as possible in each lung lobe.

### 1.2 Grouping and administration

The animals were administered for the first time within 1 h after the intratracheal nebulization with the modeling reagent (LPS), and the second time after 24 h. Specific administration groups and administration routes are shown in Table 2 below.

**Table 2 Administration groups and administration routes**

| Group number | Group | Animals | Administration routes | Administrated medications |
|---|---|---|---|---|
| G0 | Normal control group | 5 | Caudal vein injection | PBS buffer |
| G1 | Model control group | 5 | Caudal vein injection | PBS buffer |
| G2 | Administration group | 5 | Caudal vein injection | Annexin A5 (dose: 1 mg/kg) |

| | | | | |
|---|---|---|---|---|
| Note: Annexin A5 was administered as annexin A5-containing PBS buffer, and the dose was calculated by annexin A5. | | | | |

### 1.3 Sampling and end time of in vivo experiments

The day after the end of the second administration (48 h after the intratracheal nebulization with the modeling reagent).

### 1.4 Test indicators

### 1.4.1 General clinical observations

The animals were observed twice per day (once in the morning and once in the afternoon) in a quarantine period and an administration period, or observed for more times as needed. Observed contents: mental state, behavior and activity, death, respiration, secretion, fecal characters, and other abnormalities.

### 1.4.2 Body weight

Before grouped, the animals were weighed on the day of modeling and the day of administration of samples; and the animals were also weighed when they were found dead or on the brink of euthanasia.

### 1.4.3 Collection and test of bronchoalveolar lavage fluid (BALF)

Collection of samples: On the day after the second administration, all groups of animals were euthanized with CO₂, then their abdominal cavities were opened, and their abdominal arteries were cut for bleeding. The neck trachea was exposed with small scissors, a small incision was made, then the neck trachea was tied with a thread after a small tube connected to a syringe was inserted. The lung was injected with 5 ml of PBS (including 1% FBS) and aspirated three times, and this operation was repeated three times to collect BALF. The BALF was centrifuged at 4°C and 1200 rpm for 10 min, and the supernatant was stored below -70°C for later use. The precipitated cells were re-suspended with 1ML PBS, and the total cells in the BALF were counted by a blood cell counter and trypan blue staining.

Classification and test of white blood cells: The remaining cells were re-suspended for smear preparation and stained with a Wright-Giemsa staining solution to distinguish neutrophils.

### 1.4.4 Histopathological test

After the bronchoalveolar lavage fluid was collected from all the animals, the chest cavity was opened to take the right lung. The lung was infused with formalin by using an injection needle, fixed in a 10% neutral buffer formalin solution, followed by paraffin embedding, slicing, section preparation, and HE staining for pathological morphological observation of lung tissues. Professional pathologists carried out pathological testing on the lung tissues. Histopathological evaluation indicators are shown in Table 3.

**Table 3 Pulmonary pathological evaluation indicators**

| Evaluation indicator | Score | | | |
|---|---|---|---|---|
| | 0 | 1 | 2 | 3 |
| Alveolar wall thickness (3 sites) | NA | Slight | Moderate | Severe |
| Inflammatory cell infiltration (3 sites) | NA | Slight | Moderate | Severe |

### 2. Statistical analysis

Experimental data were represented by mean ± standard error tables (Mean ± S.E.M.). The data were analyzed by corresponding statistical methods such as Graphpad Prism or SPSS. P < 0.05 indicated a significant difference.

### 3. Results

### 3.1 Body weight

After LPS modeling, the body weight of each group of animals significantly decreased compared to the body weight before modeling, and there was no significant difference between the groups. The body weight of each group of animals is shown in Table 4:

**Table 4 Changes in body weight**

| Group | | Body weight (g) | | | |
|---|---|---|---|---|---|
| | | Day 0 | Day 1 | Day 2 | Day 3 |
| G1: Model control group | Mean | 255.23 | 242.88 | 228.62 | 246.20 |
| | SEM | 4.45 | 2.68 | 4.14 | 6.15 |
| G2: Administration group | Mean | 254.49 | 240.80 | 224.36 | 241.86 |
| | SEM | 5.03 | 5.39 | 4.90 | 5.86 |

### 3.2 BALF cell count

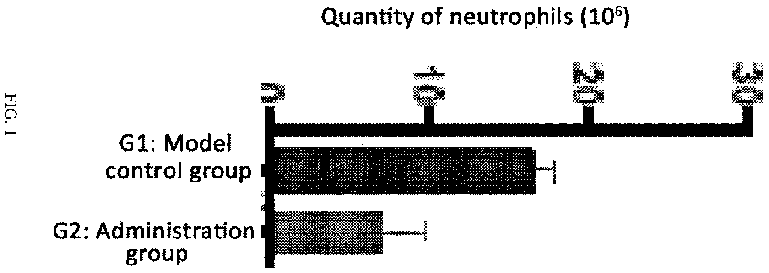

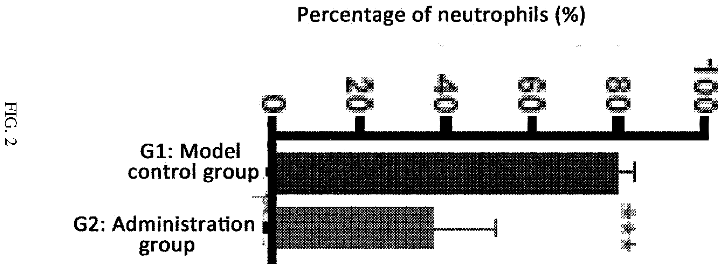

The data of BALF cell count showed a significant increase in neutrophils in the model control group G1 compared to the rats in the normal control group G0. Compared to the model control group G1, the administration group G2 significantly inhibited the count of neutrophils (Neu cells) in the BALF. Percentage data of cell typing in the BALF further showed the inhibitory effect of G2 (administration group) on the percentage count of neutrophils. Results of Neu cell counts in the BALF are shown in Table 5 and FIG. 1, and percentages of Neu cells in the BALF are shown in Table 6 and FIG. 2:

**Table 5 Neu cell counts in the BALF (*10⁶)**

| | | |
|---|---|---|
| G1: Model control group | Mean | 16.74 |
| | SEM | 1.28 |
| G2: Administration group | Mean | 7.03 |
| | SEM | 2.77 |

**Table 6 Percentages of Neu cells in the BALF (%)**

| | | |
|---|---|---|
| G1: Model control group | Mean | 79.80 |
| | SEM | 4.40 |
| G2: Administration group | Mean | 36.80 |
| | SEM | 15.00 |

### 3.3 Pathological analysis

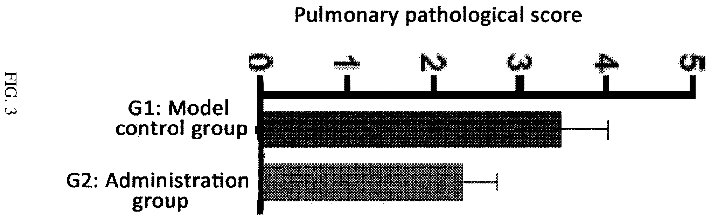

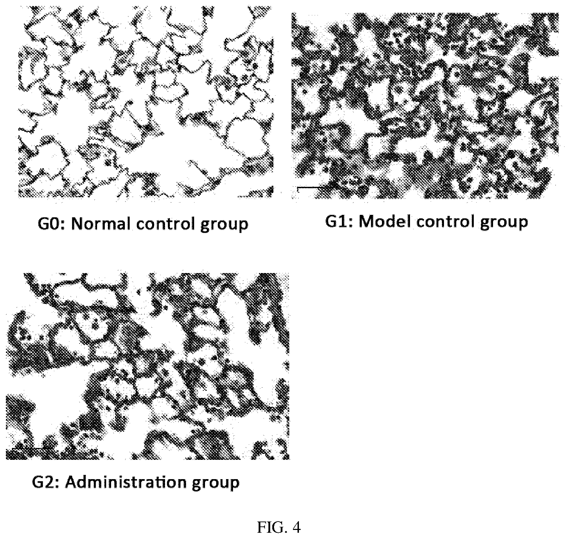

The results showed that the administration group G2 showed a significant improvement on the LPS-induced acute respiratory distress syndrome compared to the model control group G1. Pulmonary pathological scores of the model control group G1 and the administration group G2 are shown in Table 7 and FIG. 3. Typical pathological sections of lung tissues of the normal control group G0, the model control group G1, and the administration group G2 are stained as shown in FIG. 4.

**Table 7 Pulmonary pathological scores**

| | Inflammation | | | | Alveolar wall thickness | | | | | | |
|---|---|---|---|---|---|---|---|---|---|---|---|
| Group | Site d 1 | Site d 2 | Site d 3 | Mean | Site d 1 | Site d 2 | Site d 3 | Mean | Total | Mean of total | SD |
| G1-1 | 2 | 1 | 2 | 1.67 | 3 | 0 | 1 | 1.33 | 3.00 | 3.47 | 0.56 |
| G1-2 | 3 | 2 | 2 | 2.33 | 2 | 2 | 2 | 2.00 | 4.33 | | |
| G1-3 | 2 | 2 | 1 | 1.67 | 1 | 2 | 2 | 1.67 | 3.33 | | |
| G1-4 | 3 | 2 | 1 | 2.00 | 2 | 2 | 1 | 1.67 | 3.67 | | |
| G1-5 | 1 | 1 | 1 | 1.00 | 2 | 2 | 2 | 2.00 | 3.00 | | |
| G2-1 | 2 | 2 | 1 | 1.67 | 0 | 1 | 1 | 0.67 | 2.33 | 2.33 | 0.41 |
| G2-2 | 1 | 3 | 2 | 2.00 | 1 | 1 | 1 | 1.00 | 3.00 | | |
| G2-3 | 2 | 1 | 1 | 1.33 | 0 | 2 | 0 | 0.67 | 2.00 | | |
| G2-4 | 1 | 1 | 1 | 1.00 | 2 | 1 | 1 | 1.33 | 2.33 | | |
| G2-5 | 1 | 1 | 2 | 1.33 | 1 | 1 | 0 | 0.67 | 2.00 | | |

From FIG. 4, it can be seen that the lung injury in the administration group G2 was significantly improved compared to the model control group G1, indicating that annexin A5 had excellent treatment effects on the respiratory distress syndrome.

### Example 2

### 1. Preparation before experiments

### 1.1 Reagents

Test sample: Annexin A5, numbered CEFFE01, produced by Tianjin KMD Bioscience Co., Ltd., concentration 0.3 mg/mL, and stored for a long time below -20°C when unopened and 48 hours at 2°C-8°C after opened.

Modeling reagent: Lipopolysaccharides (LPS), produced by SIGMA, yellowish brown freeze-dried powder, and stored at 2°C-8°C.

Solvent: Sodium chloride injection, produced by Hunan Kelun Pharmaceutical Co., Ltd., colorless and clear liquid, and stored in a closed container.

### 1.2 Preparation and later storage of reagents

Modeling reagent: On the day of administration, an appropriate amount of lipopolysaccharides was accurately weighed, added to an appropriate amount of sodium chloride injection, shaken by vortex and blended uniformly to obtain 1 mg/mL and 5 mg/mL solutions of lipopolysaccharides.

Test sample: On the day of administration, the test sample was taken out from a -20°C refrigerator and transferred to a 2°C-8°C refrigerator or a thermal box containing an ice bag. After complete melting, the original solution was used directly.

On the day of administration, the test sample, the solvents, and the modeling reagents were transported in thermal boxes containing ice bags to animal rooms, and restored to room temperature before administration to the animals. After administration, the remaining test sample were stored in the 2°C ∼8°C refrigerator.

### 1.3 Animals

18 SPF grade Sprague-Dawley rats (SD rats) aged 8-10 weeks, weighing 321-367 g.

Source of experimental animals: Zhejiang Vital River Laboratory Animal Technology Co., Ltd; production license number for experimental animals: SCXK (Zhejiang) 2019-0001; number of experimental animal quality certificate: 20210722Aazz0619000304; production license issuing unit: Department of Science and Technology of Zhejiang Province.

Animals underwent a 10 day quarantine period, during which qualified personnel examined whole bodies of the animals and evaluated their health status. Qualified animals were approved by SD for use in this experiment.

### 1.4 Animal feeding and management

SPF grade animal rooms, SPF rat growth and breeding feed, and purified water (source: secondary reverse osmosis RO membrane filtered water prepared by our institution) supplied with drinking bottles for free drinking were used.

The animals were fed in polycarbonate plastic boxes, with no more than 5 animals per cage. The animal rooms were shielded alternately in light and dark for 12 hours.

Environmental conditions were controlled at room temperature of 20°C to 26°C, relative humidity of 40% to 70%, measured room temperature range of 22.1°C to 25.75°C, and measured humidity range of 55.35% to 71.4%.

### 2. Test content

### 2.1 Model building

Animal grouping: 18 qualified male animals were randomly divided into a normal control group, a model control group, and a test sample group according to their body weight measured before grouping, with 6 animals per group.

### Model building:

D0: The animals in the normal control group were intraperitoneally injected with sodium chloride injection (1 mL/kg), while the animals in other groups were intraperitoneally injected with lipopolysaccharides (1 mg/kg, 1 mL/kg).

D1: After 16 h (± 30 min) of intraperitoneal injection, the animals were anesthetized by inhaling isoflurane. The animals in the normal control group were nebulized with sodium chloride injection (1 mL/kg) in the trachea, while the animals in other groups were nebulized with lipopolysaccharides (5 mg/kg, 1 mL/kg) in the trachea.

Intraperitoneal injection with lipopolysaccharides (LPS): According to the recent body weight of the animals, each animal was administered with lipopolysaccharides by intraperitoneal injection with a disposable micro syringe. When the volume of lipopolysaccharides was between two graduation capacity lines, the lipopolysaccharides were pipetted according to the upper graduation value.

Intratracheal nebulization with lipopolysaccharides (LPS): Each animal was anesthetized by inhaling isoflurane and then fixed on a rat fixator placed at a 45° angle, the root of the animal's tongue was pressed by a small animal anesthesia laryngoscope to expose the glottis, a pulmonary micro liquid nebulizer needle (blunt) that extracted a quantitative solution of LPS was gently inserted into the trachea, then the piston was quickly pushed to atomize the LPS solution into the lung, the needle was quickly pulled out, and the animal was taken down from the fixator and rotated left and right with its head facing upwards to distribute LPS as evenly as possible in each lung lobe. When the volume of lipopolysaccharides was between two graduation capacity lines, the lipopolysaccharides were pipetted according to the upper graduation value.

### 2.2 Dosage design

D 1: About 3 h to 5 h after the intratracheal nebulization of the modeling reagent (LPS), the animals were administrated with the test sample or solvent according to the following table 8:

**Table 8 Dosage design for Example 2**

| Group | | Dosage (mg/kg) | Concentration (mg/mL) | Administration capacity (mL/kg) | Animal number |
|---|---|---|---|---|---|
| 1 | Normal control group | 0 | 0 | 5 | 1-6 |
| 2 | Model control group | 0 | 0 | 5 | 7-12 |
| 3 | Test sample group | 0.75 | 0.15 | 5 | 13-18 |

| | | | | | |
|---|---|---|---|---|---|
| Note: 1. The dosage was calculated by the protein in the test sample; 2. The normal control group and the model control group were intraperitoneally injected with the sodium chloride injection. | | | | | |

### Administration method

### Administration route: intravenous injection

Administration frequency and duration: On D1, the animals were administered for the first time within 3 h after the intratracheal nebulization with the modeling reagent (LPS); and on D2, the animals were administered for the second time. The interval between the two administrations was about 24 h (± 20 min).

Intravenous injection: According to the recent body weight of the animals, the dosage for each animal was extracted with a disposable syringe, and the animal was administered by tail vein injection. When the volume of the medication was between two graduation capacity lines, the medication was pipetted according to the upper graduation value.

### 3. Test

Data were collected by system generation and manual recording. The data of this experiment were represented by the "mean ± standard deviation".

### 3.1 General clinical observations

The animals were observed twice a day (once in the morning and once in the afternoon) in the quarantine period and the administration period. On the next day after administration with the modeling reagent, the animals showed abnormalities related to modeling, such as eye and nose secretions, weight loss, and depression. Other clinical observations showed no abnormalities.

### 3.2 Body weight

The animals were weighed when received, before grouping, on the day of modeling, and on the day of administration; and the animals were also weighed before euthanasia.

### 3.3 Survival test

After planned euthanasia, a rate of a quantity of live animals in each group to a total quantity of animals in each group in an experimental period was calculated.

Survival rate (%) = the quantity of live animals in a group/ the total quantity of animals in the group × 100%.

Results: During the experiment, 1 animal died in the model control group, and no animal death was found in the other groups. The survival rates of the animals in the normal control group, the model control group, and the test sample group were 100%, 83.3%, and 100%, respectively.

### 3.4 Blood gas test

### Test time: the day after the second administration (D3)

*Sample collection:* The rats were anesthetized by intraperitoneal injection with chloral hydrate (350 mg/kg, 100 mg/mL), and their abdominal midline was longitudinally incised to separate the abdominal aorta. About 0.2 mL of arterial blood was collected with an arterial blood sampler. A syringe in the blood sampler was rubbed in the palm and turned upside down for 5 seconds to ensure that the blood was never returned to the blood sampler for blood mixing treatment.

*Testing method:* After the arterial blood was collected, a needle was gently inserted into a blood injection port of a test card, and the blood was gently pushed in and filled a sample filling tube. When the blood reached an adding position, the adding of the sample was stopped, a lid was closed, and the blood automatically entered the test tube. The test card was inserted into a blood gas analyzer to wait for test results.

*Test indicators:* PO₂ (mmHg), PCO₂ (mmHg), pH, and sO₂%.

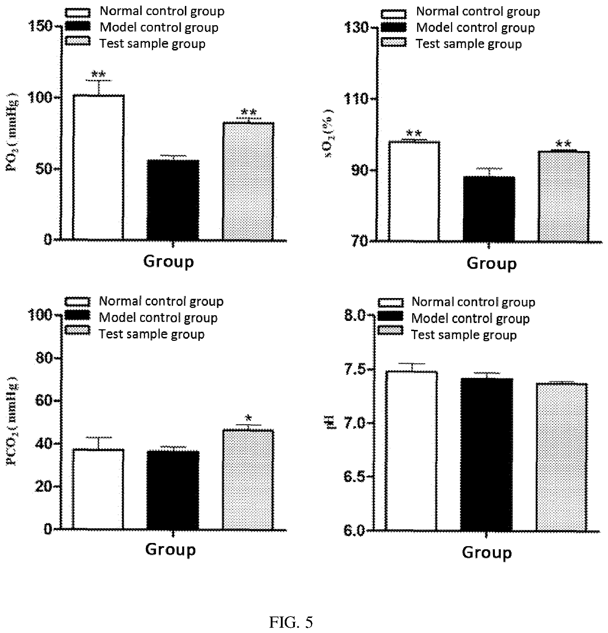

*Results:* Compared to the normal control group, the PO₂, sO₂%, and PCO₂ of the animals in the model control group decreased, where the PO₂ decreased by about 50%. However, after administration, the corresponding indicators of the animals in the test sample group all increased with statistical differences (P ≤ 0.05 or P ≤ 0.01), where the PCO₂ increased to a higher level than the normal control group, and there was no significant difference in the pH indicator. The results are shown in Table 9 and FIG. 5.

**Table 9 Effects of annexin on blood gas analysis in rats with lung injuries (x̅±SD)**

| Group | pH | PCO₂ (mmHg) | PO₂ (mmHg) | sO₂% |
|---|---|---|---|---|
| Normal control group | 7.481 ± 0.076 | 37.27 ± 5.77 | 101.5 ± 10.7** | 98.0 ± 0.6** |
| Model control group | 7.414 ± 0.124 | 36.58 ± 5.21 | 56.2 ± 7.9 | 88.2 ± 5.6 |
| Test sample group | 7.372 ± 0.049 | 46.63 ± 6.17* | 82.5 ± 8.7** | 95.5 ± 1.0** |

| | | | | |
|---|---|---|---|---|
| Note: 1. The model control group included 5 animals, while the other groups included 6 animals. Compared with the model control group, "*" represented p ≤ 0.05, and "**" represented p ≤ 0.01. | | | | |

### 3.5 Bronchoalveolar lavage fluid (BALF) test

*Sample collection:* After the animals were euthanatized, the skin and tissues of the neck and chest were incised to expose the trachea, bronchi, and lungs. The bronchi in the right lung were separated and ligated. A suture was threaded under the trachea, a 1/2 incision was made between cartilage rings of the trachea at an appropriate position under the thyroid cartilage, a trachea cannula was slowly inserted along the incision into the airway to a left bronchus, and the inserted cannula was fastened with the trachea by using the threaded suture at an appropriate position in a centripetal direction of the incision. 3 mL of PBS buffer was slowly injected with a syringe for bronchoalveolar lavage. The lavage was repeated 3 times, with each remaining for about 10 seconds. The lavage solution was collected into a centrifuge tube of appropriate capacity.

*BALF treatment:* The collected lavage solution was centrifuged at 4°C and approximately 2000 rpm for 20 min. The supernatant was stored below -60°C for later use. The precipitate was re-suspended with 1 mL of PBS buffer for count and classification of white blood cells. The supernatant after BALF centrifugation was managed as a sample by using a Watson LIMS7.5 system.

*Classification and test of white blood cells:* White blood cells in the re-suspended BALF were counted and classified by a fully automatic blood cell analyzer.

*Test of total protein content in the BALF:* At the end of the experiment, the total protein content in the BALF was tested by a total protein test kit (BCA method).

*Test of inflammatory factors in the BALF:* At the end of the experiment, the inflammatory factors (IL-1β, IL-6, IL-10, and TNF-α) in the BALF were tested by an inflammatory factor test kit.

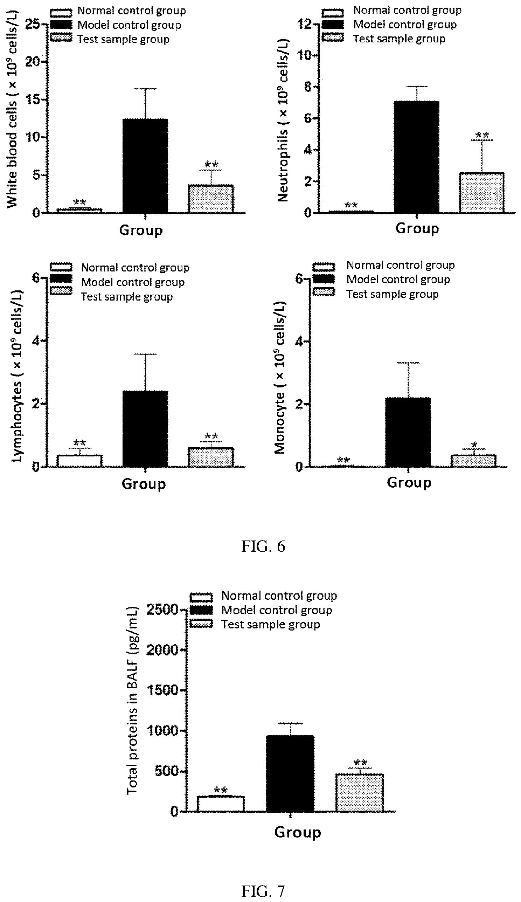

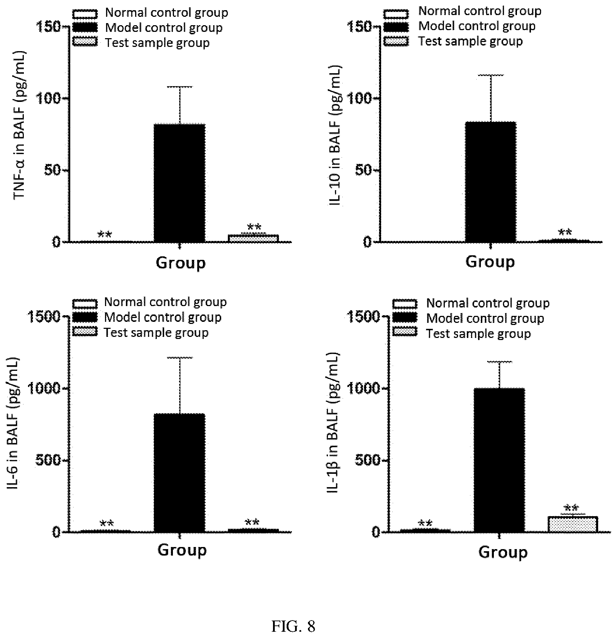

*Results:* As shown in Tables 10 and 11 and FIGs.6-8, the total number of BALF white blood cells in the animals of the model control group increased by about 10-100 times, the levels of inflammatory factors increased by about 100-900 times, and the total protein concentration increased by about 10 times after modeling, indicating a significant inflammatory response in the lung.

After administration, compared to the corresponding indicators of the animals in the model control group, the WBC (white blood cells), Neut (neutrophils), Lymph (lymphocytes), mean Mono (monocytes) count, mean total protein indicator, and mean BALF inflammatory factor (TNF-α, IL-10, IL-6, and IL-1β) indicators of the animals in the test sample group significantly decreased and showed statistical differences (P ≤ 0.05 or P ≤ 0.01), where the white blood cell count and the inflammatory factor concentration decreased to levels comparable to the normal control group, indicating a significantly excellent anti-inflammatory effect, namely, the annexin of the present invention had a comprehensive effect of inhibiting pulmonary inflammation.

**Table 10 Effects of annexin on the total number and classification of white blood cells in the BALF of rats with lung injuries (x̅±SD)**

| Group | White blood cell (× 10⁹ cells/L) | Neutrophils (×10⁹ cells/L) | Lymphocytes (×10⁹ cells/L) | Monocytes (×10⁹ cells/L) |
|---|---|---|---|---|
| Normal control group | 0.47 ± 0.21** | 0.08 ± 0.07** | 0.37 ± 0.22** | 0.02 ± 0.03** |
| Model control group | 12.35 ±4.04 | 7.06 ± 2.15 | 2.38 ± 1.19 | 2.18 ± 2.54 |
| Test sample group | 3.63 ± 2.05** | 2.54 ± 2.07** | 0.58 ± 0.22** | 0.38 ± 0.47* |

| | | | | |
|---|---|---|---|---|
| Note: 1. The model control group included 5 animals, while the other groups included 6 animals. Compared with the model control group, "*" represented p ≤ 0.05, and "**" represented p ≤ 0.01. | | | | |

**Table 11 Effects of annexin on inflammatory factors and total protein concentration in the BALF of rats with lung injuries (x̅±SD)**

| Group | TNF-α (pg/mL) | IL-10 (pg/mL) | IL-6 (pg/mL) | IL-1β (pg/mL) | Total proteins (µg/mL) |
|---|---|---|---|---|---|
| Normal control group | 0.22 ± 0.54** | 0.00 ± 0.00** | 6.91 ± 16.92** | 11.47 ± 27.84** | 186.67 ± 32.81** |
| Model control group | 81.71 ± 59.56 | 83.02 ± 74.50 | 818.16 ± 884.86 | 996.93 ± 425.54 | 927.21 ± 358.51 |
| Test sample group | 4.54 ± 4.63** | 0.92 ± 2.23** | 14.42 ± 23.92** | 107.45 ± 51.12** | 461.31 ± 187.36* |

| | | | | | |
|---|---|---|---|---|---|
| Note: 1. The model control group included 5 animals, while the other groups included 6 animals. Compared with the model control group, "*" represented p ≤ 0.05, and "**" represented p ≤ 0.01. | | | | | |

### 3.6 Test of inflammatory factors in serum

*Collection of blood sample:* About 1 mL of abdominal aortic blood of each animal was collected into a centrifuge tube containing a separation gel and a coagulant.

*Treatment of blood sample:* After collection, the blood sample was stored and transported at room temperature, and centrifuged within 2 hours under the conditions: 3000 g, room temperature, and 10 minutes. The serum after centrifugation was transferred to a new labeled centrifuge tube and divided into 2 tubes, which were temporarily stored below -60°C. At the end of the experiment, inflammatory factors (IL-1β, IL-6, IL-10, and TNF-α) in the serum were tested by a reagent kit. The blood sample was managed by the Watson LIMS7.5 system.

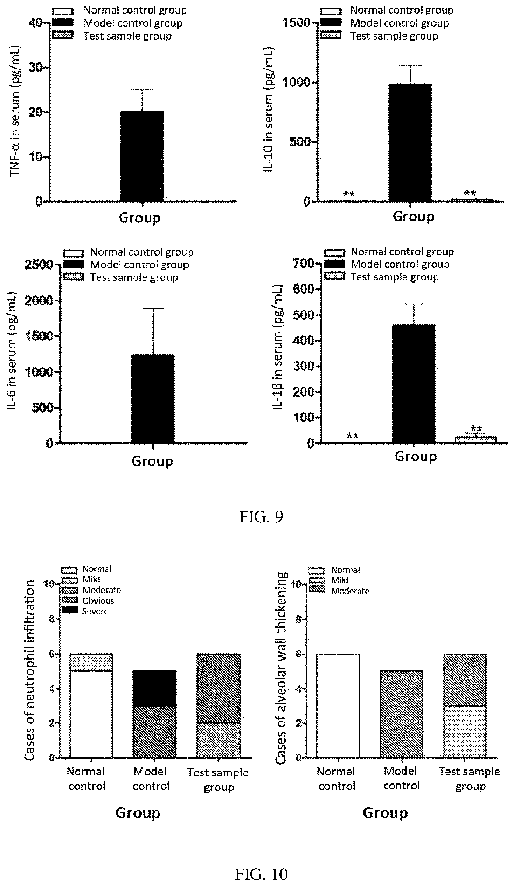

*Results:* As shown in Table 12 and FIG. 9, the levels of inflammatory factors in the serum of the animals in the model control group increased by about 100-1000 times after modeling, indicating significant inflammatory responses in the blood. After administration, compared to the corresponding indicators of the animals in the model control group, the inflammatory factors in the serum of the animals in the test sample group also significantly decreased to similar levels as the normal control group. This verified once again that the annexin of the present invention had significantly excellent anti-inflammatory effects.

**Table 12 Effects of annexin on inflammatory factors in serum of rats with lung injuries (x̅±SD)**

| Group | TNF-α (pg/mL) | IL-10 (pg/mL) | IL-6 (pg/mL) | IL-1β (pg/mL) |
|---|---|---|---|---|
| Normal control group | 0.00 ± 0.00** | 4.65 ± 5.45** | 0.00 ± 0.00** 1231.76 ± | 2.06 ± 3.22** |
| Model control group | 20.11 ± 11.20 | 980.34 ± 364.72 | 1461.02 | 461.40 ± 184.32 |
| Test sample group | 0.00 ± 0.00** | 18.35 ± 9.84** | 0.00 ± 0.00** | 15.41 ± 25.29** |

| | | | | |
|---|---|---|---|---|
| Note: 1. The model control group included 5 animals, while the other groups included 6 animals. Compared with the model control group, "**" represented p ≤ 0.01. | | | | |

### 3.6 Euthanasia of animals

According to the AVMA Guidelines for the Euthanasia of Animals: 2020 Edition (the American Veterinary Medical Association, 2020), all animals were anesthetized by intraperitoneal injection with chloral hydrate (350 mg/kg, 100 mg/mL) and euthanized by abdominal aortic bleeding on D3.

### 3.7 Histopathological test

*General observation:* All animals in each group (including dead animals) were dissected and their tissues were stored. The animals that died during non-working hours were stored in the 2°C -8°C refrigerator and dissected as soon as possible within 24 hours. After dissecting and retaining tissues, animal carcasses were disposed of as medical waste.

During the autopsy, whether the lungs, trachea, and bronchi of the animals were abnormal were observed. The middle lobe of the right lung was incised and weighed for calculating a wet/dry weight ratio. The remaining tissues and bronchi of the right lung were fixed in a 10% neutral buffered formalin solution, followed by paraffin embedding, slicing, section preparation, and HE staining for pathological morphological observation of lung tissues. Standard terminologies were used for diagnosis and classification, and the lung tissues were pathologically examined by a 5-grade method (slight, mild, moderate, obvious, and severe).

*Results:* Under the conditions of this experiment, the animals in the normal control group showed under a microscope that the lung was slightly infiltrated by inflammatory cells and bled slightly; the animals in the model control group showed under the microscope that the lung was obviously to severely infiltrated by inflammatory cells and bled mildly, with moderate proliferation of alveolar epithelial cells; and the animals in the model control group showed under the microscope that the lung was moderately to obviously infiltrated by inflammatory cells and bled mildly, with mild to moderate proliferation of alveolar epithelial cells. The degree of lung lesions in the animals of the test sample group was lower than that of the model control group. The results of the 5-grade method are shown in Table 13 and FIG. 10.

**Table 13 Effects of annexin on the degree of lung lesions in rats with lung injuries**

| Group | Degree of inflammatory cell infiltrating lesion | | | | | | Degree of alveolar wall thickening lesion | | | | | |
|---|---|---|---|---|---|---|---|---|---|---|---|---|
| | - | + | ++ | +++ | ++++ | +++++ | - | + | ++ | +++ | ++++ | +++++ |
| Normal control group | 5 | 1 | 0 | 0 | 0 | 0 | 6 | 0 | 0 | 0 | 0 | 0 |
| Model control group | 0 | 0 | 0 | 0 | 3 | 2 | 0 | 0 | 0 | 5 | 0 | 0 |
| Test sample group | 0 | 0 | 0 | 2 | 4 | 0 | 0 | 0 | 3 | 3 | 0 | 0 |

| | | | | | | | | | | | | |
|---|---|---|---|---|---|---|---|---|---|---|---|---|
| Note: The lesions were graded by the 5-grade method, - = no lesion; + = slight; ++ = mild; +++ = moderate; ++++ = obvious; +++++ = severe. | | | | | | | | | | | | |

### 3.8 Wet/dry ratio of the lung

Foreign matters such as blood and tissue fluid on the surface of the middle lobe tissues of the right lung were wiped with a paper towel, the right lung was weighed, then the middle lobe tissues of the right lung were placed in a 60°C oven for 72 h, the right lung was weighed again, and the wet/dry weight ratio of the lung was calculated.

Wet/dry ratio of the lung = wet weight/ dry weight of the middle lobe of the right lung

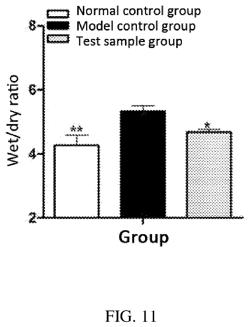

*Results:* The wet/dry ratio of the lung of the animals in the test sample group decreased, as shown in Table 14 and FIG. 11.

**Table 14 Effects of annexin on wet/dry ratio of the lung of rats with lung injuries (x̅±SD)**

| Group | Wet/dry ratio of the lung |
|---|---|
| Normal control group | 4.28 ± 0.31** |
| Model control group | 5.34 ± 0.43 |
| Test sample group | 4.68 ± 0.08* |

| | |
|---|---|
| Note: 1. Each group included 6 animals. Compared with the model control group, "*" represented p ≤ 0.05, and "**" represented p ≤ 0.01. | |

### Discussion

The annexin of the present invention had excellent treatment effects on rats with lung injury, reflected in:
(1) The survival rate was increased;
(2) In the blood gas analysis, PO₂ (mmHg) and sO₂% were increased compared to the model control group;
(3) In the BALF test, the quantity of white blood cells significantly decreased to a level comparable to that of the normal control group; the quantity of total proteins also significantly decreased; the concentration of inflammatory factors significantly decreased to a normal control level;
(4) The inflammatory factors in serum were significantly reduced;
(5) Severe and moderate lesions in lung tissues were effectively reduced, inflammatory cell infiltrating lesions in all rats changed from severe and obvious to obvious and moderate, and alveolar wall thickness in half of the rats changed from moderate to mild;
(6) The wet/dry ratio of lung was reduced.

In summary, the annexin of the present invention treated the rats with lung injuries (acute respiratory distress syndrome) from multiple aspects and multiple perspectives and achieved excellent treatment effects.

All documents mentioned in the present invention are cited as references in the present application, just as each document is individually cited as a reference. Moreover, it should be understood that, after reading the above teaching contents of the present invention, those skilled in the art can make various alterations or modifications to the present invention, and these equivalent forms also fall within the scope defined by the appended claims of the present application.

## Claims

1. An use of an annexin, wherein for preparation of a composition or a formulation, the composition or the formulation has one or more uses selected from the following group consisting of: (i) preventing and/or treating lung injury; (ii) reducing a quantity of neutrophils; (iii) increasing blood gas PO₂ and/or sO₂% indicators; (iv) reducing pulmonary inflammation; (v) reducing inflammatory factors in serum; (vi) improving the degree of lung lesions; and/or (vii) decreasing a wet/dry ratio of a lung.

2. The use of claim 1, wherein the annexin comprises an annexin A5.

3. The use of claim 1, wherein the lung injury is selected from the following group consisting of: an acute lung injury, a respiratory distress syndrome, or a combination thereof.

4. The use of claim 1, wherein the lung injury includes a lung injury caused by an increase in neutrophils; and/or
the lung injury includes a lung injury caused by a cytokine storm.

5. The use of claim 1, wherein the preventing and/or treating lung injury includes preventing and/or treating lung injury by reducing quantity of neutrophils.

6. The use of claim 1, wherein the preventing and/or treating lung injury includes one or more features selected from the following group consisting of:
(i) increasing a survival rate;
(ii) increasing blood gas PO₂ and/or sO₂% indicators;
(iii) reducing pulmonary inflammation;
(iv) reducing inflammatory factors in serum;
(v) improving degree of lung lesions;
(vi) decreasing wet/dry ratio of lung.

7. The use of claim 4, wherein the neutrophils comprise neutrophils in lung; and/or
the cytokine storm comprises a cytokine storm in lung.

8. The use of claim 1, wherein the reducing quantity of neutrophils refers to reducing quantity of neutrophils in a subject or reducing neutrophil aggregation in lung of a subject.

9. The use of claim 8, wherein the subject is a human with an increase in quantity of neutrophils; preferably, the increase in the quantity of neutrophils refers to a higher quantity of neutrophils A1 in the subject, compared with a quantity of neutrophils A0 in a normal subject; preferably, A1/A0 ≥ 1.1, preferably ≥ 1.2, more preferably ≥ 1.3, more preferably ≥ 1.4, more preferably ≥ 1.5, more preferably ≥ 1.6, more preferably 1.8, more preferably ≥ 2, more preferably 3, more preferably ≥ 5, more preferably ≥ 8.

10. The use of claim 6, wherein the reducing pulmonary inflammation is performed by one or more ways selected from the following group consisting of:
(iii-1) reducing pulmonary inflammatory factors;
(iii-2) reducing pulmonary white blood cells;
(iii-3) reducing pulmonary total proteins.

11. The use of claim 10, wherein the pulmonary inflammatory factors comprise TNF-α, IL-10, IL-6, and/or IL-1β.

12. The use of claim 10, wherein the pulmonary white blood cells comprise white blood cells, neutrophils, lymphocytes, monocytes.

13. The use of claim 10, wherein the reducing pulmonary inflammatory factors refers to a lower quantity of pulmonary inflammatory factors (E1) of an administrated subject, compared with a quantity of inflammatory factors (E0) of a lung injury subject; and preferably, E0/E1 ≥ 10.

14. The use of claim 10, wherein the reducing white blood cells refers to a lower quantity of white blood cells (F1) of the administrated subject, compared with a quantity of white blood cells (F0) of lung injury subject; and preferably, F0/F1 ≥ 3.

15. The use of claim 10, wherein the reducing pulmonary total proteins refers to a lower quantity of total proteins (H1) of the administered subject, compared with a quantity of total proteins (H0) of lung injury subject; and preferably, H0/H1 ≥ 2.

16. The use of claim 10, wherein the inflammatory factors in serum comprise TNF-α, IL-10, IL-6, and/or IL-1β.

17. The use of claim 10, wherein the reducing the inflammatory factors in serum refers to a lower quantity of inflammatory factors (J1) in the serum of the subject, compared with a quantity of inflammatory factors (10) in lung injury subject; and preferably J0/J1 & 20.

18. The use of claim 1, wherein the composition or the formulation further comprises other medications for preventing and/or treating lung injury; and
preferably, the other medications for preventing and/or treating lung injury are selected from the following group consisting of: a glucocorticoid, a vitamin, or a combination thereof.

19. The use of claim 1, wherein dosage form of the composition or the formulation is powder, granule, capsule, injection, tincture, oral liquid, tablet, buccal tablet, dry powder inhalation, aerosol, or spray.

20. The use of claim 19, wherein the injection is an intravenous injection, an intramuscular injection, a subcutaneous formulation, a respiratory administration formulation.
